# EUROPEAN PATENT APPLICATION

(11) **EP 3 949 958 A1**
(43) Date of publication of application: **09.02.2022**
(21) Application number: 20189451.6
(22) Date of filing: 04.08.2020
(51) Int. Cl.: A61K 31/21, A61K 31/34, A61K 31/35, A61K 31/5355, A61K 31/655, A61K 33/26, A61P 27/02, A61P 43/00

(54) **COMPOUND FOR THE TREATMENT AND PROPHYLAXIS OF A LIPOFUSCIN-ASSOCIATED DISEASE**

(71) Applicant: Eberhard Karls Universität Tübingen Medizinische Fakultät, 72074 Tübingen (DE)
(72) Inventor: Schraermeyer, Ulrich, 72379 Hechingen (DE)
(74) Representative: Witte, Weller & Partner Patentanwälte mbB

(57) **Abstract**

The present invention relates to a compound and a pharmaceutical composition for use in the treatment and/or prophylaxis of a lipofuscin-associated disease and/or a disease associated with aged oxidized melanin of a living being. It also relates to a method for the production of a pharmaceutical composition for the treatment and/or prophylaxis of a lipofuscin-associated disease and/or a disease associated with aged oxidized melanin of a living being. It further relates to a compound for use in the removal of lipofuscin and/or aged oxidized melanin from a living being. It finally relates to a method for the therapeutic and/or prophylactic treatment of a living being affected by or suspected of being affected by a lipofuscin-associated disease and/or a disease associated with aged oxidized melanin.

## Description

The present invention relates to a compound and a pharmaceutical composition for use in the treatment and/or prophylaxis of a lipofuscin-associated disease and a disease associated with aged oxidized melanin of a living being. It also relates to a method for the production of a pharmaceutical composition for the treatment and/or prophylaxis of a lipofuscin-associated disease and a disease associated with aged oxidized melanin of a living being. It further relates to a compound for use in the removal of lipofuscin and aged oxidized melanin from a living being. It finally relates to a method for the therapeutic and/or prophylactic treatment of a living being affected by or suspected of being affected by a lipofuscin-associated disease and a disease associated with aged oxidized melanin.

### FIELD OF THE INVENTION

The present invention relates to the field of biosciences, in particular the field of pharmaceutically active agents.

### BACKGROUND OF THE INVENTION

Lipofuscin is a general term to describe fine yellow-brown pigment granules composed of lipid-containing residues of lysosomal digestion. It is considered to be one of the aging or "wear-and-tear" pigments, found in the liver, kidney, heart muscle, retina, adrenals, nerve cells, and ganglion cells. It is specifically arranged around the nucleus, and is a type of lipochrome.

In its broadest sense, the accumulation of critical amounts of lipofuscin is pathologic in any tissue, but especially so in the tissues of the CNS where the loss of cell function through lipofuscin is particularly apparent.

The composition of lipofuscin is complex and still under investigation. It appears to be the product of the oxidation of unsaturated fatty acids and may be symptomatic of membrane damage, or damage to mitochondria and lysosomes. Aside from a large lipid content, lipofuscin is known to contain sugars and metals, including mercury, aluminum, iron, copper and zinc. Lipofuscin is also accepted as consisting of oxidized proteins (30-70%) as well as lipids (20-50%).

In the eye, one important and well characterized component of lipofuscin is the flurophore N-retinylidene-N-retinylethanolamine (A2E), a byproduct of the visual cycle. It can be detected histologically by its autofluorescence properties. The origin of lipofuscin in the retinal pigment epithelium (RPE) is still under debate; see Kennedy et al. (1995), Lipofuscin of the Retinal Pigment Epithelium: A Review', Eye (London, England), 9 (Pt 6), pp. 763-771.

The accumulation of lipofuscin-like material may be the result of an imbalance between formation and disposal mechanisms: Such accumulation can be induced in rats by administering a protease inhibitor (leupeptin); after a period of three months, the levels of the lipofuscin-like material return to normal, indicating the action of a significant disposal mechanism. However, this result is controversial, as it is questionable if the leupeptin-induced material is true lipofuscin. There exists evidence that "true lipofuscin" is not degradable *in vitro;* whether this holds in vivo over longer time periods is not clear.

Lipofuscinoses and lipofuscinopathies are diseases characterized by high levels of lipofuscin deposits as a result of aging, or metabolic defects. Lipofuscin-associated degenerative diseases of the eye have in common that lipofuscin is accumulated in the cells of the RPE. Such diseases include retinal lipofuscinopathy, age-related macular degeneration (AMD), Stargardt disease or Morbus Stargardt, Best disease or vitelliform macular dystrophy and subpopulations of Retinitis pigmentosa (RP).

Retinal lipofuscinopathy is a general term describing the accumulation of lipofuscinin in the RPE which may result in degeneration of the retina and vision loss.

AMD is a medical condition which may result in blurred or no vision in the center of the visual field. The pathogenesis of age-related macular degeneration is not well known, although some theories have been put forward, including oxidative stress, mitochondrial dysfunction, and inflammatory processes. The imbalance between the production of damaged cellular components and degradation leads to the accumulation of harmful products, for example, intracellular lipofuscin and extracellular drusen. Incipient atrophy is demarcated by areas of retinal pigment epithelium (RPE) thinning or depigmentation that precede geographic atrophy in the early stages of AMD. In advanced stages of AMD, atrophy of the RPE (geographic atrophy) and/or development of new blood vessels (neovascularization) result in the death of photoreceptors and central vision loss. In the dry (nonexudative) form, cellular debris called drusen accumulates between the retina and the choroid, causing atrophy and scarring to the retina. In the wet (exudative) form, which is more severe, blood vessels grow up from the choroid (neovascularization) behind the retina which can leak exudate and fluid and also cause hemorrhaging.

Morbus Stargardt or Stargardt disease is the most common inherited single-gene retinal disease. It usually has an autosomal recessive inheritance caused by mutations in the ABCA4 gene. Rarely, it has an autosomal dominant inheritance due to defects with ELOVL4 or PROM1 genes. It is characterized by macular degeneration that begins in childhood, adolescence or adulthood, resulting in progressive loss of vision. In the most common form of Stargardt disease caused by mutations in the ABCA4 gene, STGD1, the genetic defect causes malfunction of the ATP-binding cassette transporter (ABCA4) protein of the visual phototransduction cycle. Defective ABCA4 leads to improper shuttling of vitamin A throughout the retina, and accelerated formation of toxic vitamin A dimers (also known as bisretinoids), and associated degradation byproducts. Vitamin A dimers and other byproducts are widely accepted as the cause of STGD1. When vitamin A dimers and byproducts damage the retinal cells, lipofuscin in the retinal pigmented epithelium of the retina appear, as a reflecting such damage. In STGD4, a butterfly pattern of dystrophy is caused by mutations in a gene that encodes a membrane bound protein that is involved in the elongation of very long chain fatty acids (ELOVL4). In STGD3, a rare dominant form of Stargardt disease caused by mutations in the ELOVL4 gene, a butterfly pattern of dystrophy is caused by mutations in a gene that encodes a membrane bound protein that is involved in the elongation of very long chain fatty acids.

Vitelliform macular dystrophy or Best disease, is an irregular autosomal dominant eye disorder and a retinal lipofuscinosis which can cause progressive vision loss, caused by mutations in the RDS and VMD2 genes. This disorder affects the retina, specifically cells in a small area near the center of the retina called the macula. Mutations in the VMD2 gene are responsible for Best disease. Changes in either the VMD2 or RDS gene can cause the adult-onset form of vitelliform macular dystrophy; however, less than a quarter of cases result from mutations in these two genes. In most cases, the cause of the adult-onset form is unknown.

RP is a genetic disorder of the eyes that causes loss of vision. It is generally inherited from a person's parents. Mutations in one of more than 50 genes are involved. The underlying mechanism involves the progressive loss of rod photoreceptor cells in the back of the eye. This is generally followed by loss of cone photoreceptor cells. Abnormal levels of lipofuscin accumulation are observed in more than one-half of RP patients.

Lipofuscin-associated diseases are also found in other tissues. In the peripheral nervous system, abnormal accumulation of lipofuscin or lipofuscinosis, respectively, are associated with a family of neurodegenerative disorders - neuronal ceroid lipofuscinoses, the most common of these is Batten disease. Also, pathological accumulation of lipofuscin is implicated in Alzheimer's disease, Parkinson's disease, amyotrophic lateral sclerosis, certain lysosomal diseases, acromegaly, denervation atrophy, lipid myopathy, chronic obstructive pulmonary disease, and centronuclear myopathy. Accumulation of lipofuscin in the colon is the cause of the condition melanosis coli. Also, pathological accumulation of lipofuscin is implicated in Alzheimer's disease, Parkinson's disease, amyotrophic lateral sclerosis, certain lysosomal diseases, acromegaly, denervation atrophy, lipid myopathy, chronic obstructive pulmonary disease, and centronuclear myopathy. In Parkinson's disease the pigmented neurons of the Substantia nigra lose their melanin granules before they degenerate. The cause for this phenomenon is not known but it has been related to the pathomechanism of Morbus parkinson. Accumulation of lipofuscin in the colon is the cause of the condition melanosis coli.

Calorie restriction, vitamin E, and increased glutathione appear to reduce the production of lipofuscin in an experimental setup. The nootropic drug piracetam appears to reduce accumulation of lipofuscin in the brain tissue of rats. Other possible treatments involve the administration of centrophenoxine, acetyl-L-carnitine, Ginkgo biloba, dimethylethanolamine.

Further, the use a tetrahydropyridoether is discussed to remove lipofuscin from retinal pigment epithelial cells; see Julien and Schraermeyer (2012), Lipofuscin can be removed from the retinal pigment epithelium of monkeys, Neurobiol. Aging, 33 (10), pp. 2390-2397.

The WO 2015/121441 discusses the use of NO generators for the treatment of a lipofuscin-associated disease of the eye.

The tips of the photoreceptor outer segments are shed daily and phagocytosed by the retinal pigment epithelium. This is a very important process and a challenge for the postmitotic RPE cells that have to fulfill the degradation of the rod outersegments which additionally contain toxic bisretinoids during lifespan. The membrane of the phagosomes contains NADPase and during phagocytosis superoxide is released into the lumen of the phagosome. Additionally other radicals and reactive molecules are formed or are present during phagocytosis within the phagosomes. Among them are OH (hydroxyl radical), O²⁻, H₂O₂, singlet oxygen, riboflavin and Fe²⁺⁺. The latter induce the hazardous Fenton reaction.

Melanin has unusual redox properties. It can both generate and absorb radicals. Melanin can change any energy into electric energy (electron flow). Even gamma radiation can be converted by melanin into chemical energy for growth by radiotrophic fungi detected for example in Tschernobyl. Melanin is an O²⁻ donator and serves as a naturally occurring biological source of electrons to power biochemical reactions. Electrons degrade retinoids and bisretinoids by electrolysis. Melanosomes are the organelles in which the melanin is formed are specialized lysosomes and contain many lysosomal enzymes. The natural function of lysosomes is the degradation of molecules. Melanosomes of the RPE in mammals are only formed before birth and lack any turnover during life.

Therefore, aged oxidized melanin may accumulate in the retinal pigment epithelium resulting in a disease associated therewith.

So far there is no therapy available against lipofuscin-associated diseases or diseases associated with aged oxidized melanin, which did prove helpful in practice. In particular, most of the currently used or proposed pharmacological substances are characterized by severe side effects, such as cytotoxicity, which render them unsuitable for a use in human patients.

Against this background it is an object underlying the invention to provide a compound for use in the treatment and/or prophylaxis of a lipofuscin-associated disease and a disease associated with aged oxidized melanin by means of which the problems of the current therapies or compounds are avoided or at least reduced. In particular, a compound should be provided which is predestined for a therapeutic and/or prophylactic treatment of a living being affected by or suspected of being affected by a lipofuscin-associated disease and a disease associated with aged oxidized melanin.

The present invention satisfies these and other needs.

### SUMMARY OF THE INVENTION

This object is met by a compound for use in the treatment and/or prophylaxis of a lipofuscin-associated disease and/or a disease associated with aged oxidized melanin of a living being, which is selected from the group consisting of: 3-morpholinosydnonimine (SIN-1), Rose Bengal (Acid Red 94), DEA-NO, isosorbide dinitrate (ISDN), isosorbide mononitrate (ISMN), sodium nitroprusside (SNP), pentaerythritol tetranitrate (PETN), nitroglycerin (NG), iron (Fe), agents performing redox reactions with melanosomes, agents effecting chemiexcitation of melanin, and agents forming cyclobutane pyrimidine dimers (CPDs).

The inventor has surprisingly found that the indicated compounds allow an effective treatment and/or prophylaxis of a lipofuscin-associated disease or a disease associated with aged oxidized melanin due to their ability to remove excessive lipofuscin or aged oxidized melanin. This finding could not be expected because some of the identified compounds are described in the art as being cytotoxic, e.g. SIN-1, see Gergel et al. (1995), Increase Cytotoxicity of 3-Morpholinosydnonimine to HepG2 Cells in the Presence of Superoxide Dismutase, The Journal of Biological Chemistry, Vol. 270, No. 36, pp. 20922-20929. However, the inventor could not confirm such observations, in particular when the compounds according to the invention are used for the treatment of a lipofuscin-associated disease of the eye.

According to the invention, "lipofuscin-associated diseases" refer to any kind of disease of an individual where, in comparison to a healthy reference individual, the lipofuscin level is altered. Such diseases include lipofuscinoses and lipofuscinopathies which are characterized by high levels of lipofuscin deposits, for instance as a result of aging, or metabolic defects. They include the diseases mentioned at the outset. It also includes melanolipofuscin-associated diseases. Lipofuscin-associated diseases of the eye have in common that lipofuscin is accumulated in the cells of the retinal pigment epithelium (RPE).

According to the invention a "disease associated with aged oxidized melanin" refers any kind of disease of an individual where, in comparison to a healthy reference individual, aged oxidized melanin is accumulated above average, preferably in the retinal pigment epithelium. Aged oxidized melanin is a characteristic of the aging eye in general and in particular of the degeneration of the macula; see Rozanowski et al. (2008), Human RPE melanosomes protect from photosensitized and iron-mediated oxidation but become pro-oxidant in the presence of iron upon photodegradation, Investigative Ophthalmology & Visual Science, Vol. 49, No. 7, pp. 2838-2847.

According to the invention, a "living being" refers to any animal, including mammals, or to a human being.

3-morpholinosydnonimine or SIN-1, also referred to as linsidomine (CAS number 33876-97-0 or 16142-27-1 (hydrochloride)) is a metabolite of the antianginal drug molsidomine and known for its vasodilatory activity. It, in particular, includes the chloride salt thereof. It acts by releasing NO from the endothelial cells non-enzymatically. It also hyperpolarizes the cell membrane through influencing the sodium-potassium pump and thereby rendering it less responsive to adrenergic stimulation. SIN-1 is known for its therapeutic properties in the treatment of erectile dysfunction; see Lemaire and Buvat (1998), [Erectile response to intracavernous injection of linsidomine in 38 impotent patients. Comparison with prostaglandin E1; in French], Prog. Urol. 8(3), pp. 388-391.

Rose bengal or Acid Red 94 (4,5,6,7-tetrachloro-2',4',5',7'-tetraiodofluorescein; CAS number 4159-77-7) is a stain. Its sodium salt is commonly used in eye drops to stain damaged conjunctival and corneal cells and thereby identify damage to the eye. The stain is also used in the preparation of Foraminifera for microscopic analysis, allowing the distinction between forms that were alive or dead at the time of collection. A form of rose bengal is also being studied as a treatment for certain cancers and skin conditions. The cancer formulation of the drug, known as PV-10, is currently undergoing clinical trials for melanoma, breast cancer, neuroendocrine tumors, and eczema and psoriasis.

DEA-NO or diethylamine NONOate (diethylammonium (Z)-1-(N,N-diethylamino)diazen-1-ium-1,2-diolate; CAS number 372965-00-9) is a is a NO donor. It spontaneously dissociates in a pH-dependent, first-order process with a half-life of 2 minutes and 16 minutes at 37°C and 22-25°C, pH 7.4, respectively, to liberate 1.5 moles of NO per mole of parent compound.

Isosorbide dinitrate (ISDN) (3,6-bisnitrooxyhexahydrofuro[3,2-b]-furan; CAS number 87-33-2) is a medication used for heart failure, esophageal spasms, and to treat and prevent chest pain from not enough blood flow to the heart. It has been found to be particularly useful in heart failure due to systolic dysfunction together with hydralazine in black people. Isosorbide dinitrate is converted to nitric oxide (NO).

Isosorbide mononitrate (ISMN) (CAS number 16051-77-7) is a nitrate-class drug used for heart-related chest pain (angina), heart failure, and esophageal spasms. It is a NO donor that releases nitric oxide through enzymatic metabolism and has a vasodilating effect. It tends to group blood vessels in groups of the same size and thereby increase blood flow, because the vessels are expanded.

Sodium nitroprusside (SNP) (CAS number 13755-38-9) is a medication used to lower blood pressure. Sodium nitroprusside works by increasing NO levels in the blood, it breaks down in circulation to release NO.

Pentaerythritol tetranitrate (PETN) also known as PENT, PENTA, TEN, corpent, or penthrite (or, rarely and primarily in German, as nitropenta) (CAS number 78-11-5) is used as a vasodilator drug to treat certain heart conditions, such as for management of angina. It works by releasing NO in the body.

Nitroglycerin (NG) also known as nitroglycerine, trinitroglycerin (TNG), nitro, glyceryl trinitrate (GTN), or 1,2,3-trinitroxypropane (CAS number 55-63-0) has been used as a potent vasodilator to treat heart conditions, such as angina pectoris and chronic heart failure. These beneficial effects are due to nitroglycerin being converted to NO.

"Chemiexcitation" is a process that generates an electronically excited molecule by a chemical reaction of reactants in their ground state. According to the invention an agent effecting chemiexcitation of melanin is preferred.

None of the above-captioned compounds has been described so far for the treatment and/or prophylaxis of a lipofuscin-associated disease or a disease associated with aged oxidized melanin.

It has been shown that the radicals NO and superoxide (O₂⁻) form the powerful oxidant peroxynitrite (ONOO⁻). A fragment of the skin pigment melanin is then oxidized, exciting an electron to an energy level so high that it is rarely seen in biology. This process of chemically exciting electrons, termed "chemiexcitation" results in degradation of melanin and it occurs in melanocytes. In melanocytes, the energy transfers radiationlessly to DNA, inducing cyclobutane pyrimidine dimers (CPDs). Chemiexcitation is a new source of genome instability, and it calls attention to endogenous mechanisms of genome maintenance that prevent electronic excitation or dissipate the energy of excited states. Chemiexcitation of melanin also triggers pathogenesis and carciogenesis.

It was, therefore, even more surprising that the NO generators provided by the invention are appropriate therapeutic and prophylactic agents against lipofuscin-associated diseases or diseases associated with aged oxidized melanin.

The object underlying the invention is herewith completely solved.

In an embodiment the compound of the invention is configured for a use in the treatment and/or prophylaxis of a lipofuscin-associated disease of the eye of a living being or a disease of the eye associated with aged oxidized melanin.

This embodiment of the invention recognized in an advantageous manner the finding of the inventor that the indicated compounds allow an effective treatment and/or prophylaxis of a lipofuscin-associated disease of the eye or a disease of the eye associated with aged oxidized melanin, due to their ability to remove excessive lipofuscin and aged oxidized melanin from the eye, in particular from the retinal pigment epithelium (RPE).

In an embodiment the compound according to the invention is configured for an injection into the living being.

This measure has the advantage that a dosage form is provided with allows an effective administration to the living being. "Injection" includes all kinds of parenteral administrations, e.g. subcutaneous, intramuscular, intravenous, intraperitoneal, intraosseous, intracardiac, intraarticular, intracavernous, intravitreal injection, etc.

In another embodiment the compound according to the invention is configured for an intravenous and/or intravitreal injection into the living being.

This measure has the advantage that a dosage form is provided which allows a rapid availability and accumulation of the compound at the side of action, e.g. the lipofuscin deposits or deposits of aged oxidized melanin.

In another embodiment of the invention the lipofuscin-associated disease of the eye is selected from the group consisting of: retinal lipofuscinopathy, age-related macular degeneration (AMD), preferably in its dry form (dry AMD), Morbus Stargardt, vitelliform macular dystrophy (Best disease), and Retinitis pigmentosa.

This measure has the advantage that the compound according to the invention is adapted for the treatment or prophylaxis of the most relevant lipofuscin-associated diseases of the eye.

Another subject-matter of the invention relates to a pharmaceutical composition for the treatment and/or prophylaxis of a lipofuscin-associated disease and/or a disease associated with aged oxidized melanin of a living being, preferably a lipofuscin-associated disease of the eye or a disease of the eye associated with aged oxidized melanin, comprising the compound of according to the invention and a pharmaceutically acceptable carrier.

Pharmaceutically acceptable carriers are well known to the skilled person. They allow a proper formulation of the active agent and serve to improve the selectivity, effectiveness, and/or safety of drug administration. Pharmaceutically acceptable carriers include, without being limited thereto, solvents, fillers, binders, lubricants, stabilizers, surfactants, suspensions, thickeners, emulsifiers, preserving agents, liposomes, micelles, microspheres, nanoparticles, etc. suitable for the particular form of dosage. Except for cases, when the medium of conventional carriers is incompatible with the active ingredient, for example, upon occurrence of any undesirable biological effects or other adverse interactions with any other ingredient(s) of the pharmaceutical composition, the use of such compositions falls within the scope of this invention. Materials that can serve as pharmaceutically acceptable carriers include, but are not limited to, monosaccharides and oligosaccharides, as well as derivatives thereof; malt, gelatin; talc; excipients such as: cocoa butter and suppository waxes; oils, such as peanut oil, cottonseed oil, safflower oil, sesame oil, olive oil, corn oil and soybean oil; glycols such as propylene glycol; esters, such as ethyl oleate and ethyl laurate; agar; buffering agents, such as magnesium hydroxide and aluminium hydroxide; alginic acid; pyrogen-free water; isotonic saline, Ringer's solution; ethyl alcohol and phosphate buffer solutions. In addition, the composition may contain other non-toxic compatible lubricants, for example sodium lauryl sulfate and magnesium stearate, as well as coloring agents, parting liquids, film formers, sweeteners, flavoring additives and flavorants, preserving agents and antioxidants.

In an embodiment of the invention the pharmaceutical preparation can include any of the compounds SIN-1, Rose Bengal, DEA-NO, ISDN, ISMN, SNP, PETN, NG, Fe, agents performing redox reactions with melanosomes, agents effecting chemiexcitation of melanin, and agents forming CPDs as the only active agent. It can also comprise combinations thereof. However, in another embodiment it can also include additional active agents against a lipofuscin-associated disease and/or a disease associated with aged oxidized melanin. Pharmaceutical compositions according to the present invention can, therefore, be used both as monotherapy and administered to a patient in need of appropriate therapy in combination with one or more other therapeutic agents.

The features, advantages and characteristics of the compound according to the invention apply likewise to the pharmaceutical composition according to the invention.

Another subject-matter of the present invention relates to a method for the production of a pharmaceutical composition for the treatment and/or prophylaxis of a lipofuscin-associated disease and/or a disease associated with aged oxidized melanin of a living being, preferably a lipofuscin-associated disease of the eye and/or disease of the eye associated with aged oxidized melanin, comprising the following steps:
(i) Providing the compound according to the invention, and
(ii) Formulating said compound into a pharmaceutically acceptable carrier.

The features, advantages and characteristics of the compound and pharmaceutical composition according to the invention apply likewise to the method for production according to the invention.

Another subject-matter of the present invention relates to a compound for use in the removal of lipofuscin and/or aged oxidized melanin from a living being, preferably from the eye of the living being, which is selected from the group consisting of: 3-morpholinosydnonimine (SIN-1), Rose Bengal (Acid Red 94), DEA-NO, isosorbide dinitrate (ISDN), isosorbide mononitrate (ISMN), sodium nitroprusside (SNP), pentaerythritol tetranitrate (PETN), nitroglycerin (NG), iron (Fe), agents performing redox reactions with melanosomes, agents effecting chemiexcitation of melanin, and agents forming cyclobutane pyrimidine dimers (CPDs).

The features, advantages and characteristics of the compound and pharmaceutical composition for use in the treatment and/or prophylaxis of a lipofuscin-associated disease according to the invention apply likewise to the compound for use in the removal of lipofuscin and/or aged oxidized melanin according to the invention.

In an embodiment of the compound according to the invention said compound is configured for a removal of the lipofuscin and/or aged oxidized melanin from the retinal pigment epithelium of the living being.

This measure has the advantage that the compound is further developed for an ophthalmologic use. It is optimized to remove the lipofuscin and/or aged oxidized melanin from the site of accumulation in the eye. The compound, therefore, is improved in its effectiveness.

Another subject-matter of the invention relates to method for the therapeutic and/or prophylactic treatment of a living being affected by or suspected of being affected by a lipofuscin-associated disease and/or disease associated with aged oxidized melanin, preferably a lipofuscin-associated disease of the eye and/or a disease of the eye associated with aged oxidized melanin, comprising the following steps:
(i) Providing the compound or the pharmaceutical composition according to the invention, and
(ii) administering said compound and/or said composition into said living being.

The features, advantages and characteristics of the compound and pharmaceutical composition according to the invention apply likewise to the method for production according to the invention.

It is to be understood that the before-mentioned features and those to be mentioned in the following cannot only be used in the combination indicated in the respective case, but also in other combinations or in an isolated manner without departing from the scope of the invention.

### EMBODIMENTS

The invention is now further explained by means of embodiments resulting in additional features, characteristics and advantages of the invention. The embodiments are of pure illustrative nature and do not limit the scope or range of the invention. The features mentioned in the specific embodiments are features of the invention and may be seen as general features which are not applicable in the specific embodiment but also in an isolated manner in the context of any embodiment of the invention.

The invention is now further described and explained in further detail by referring to the following non-limiting examples and figures:

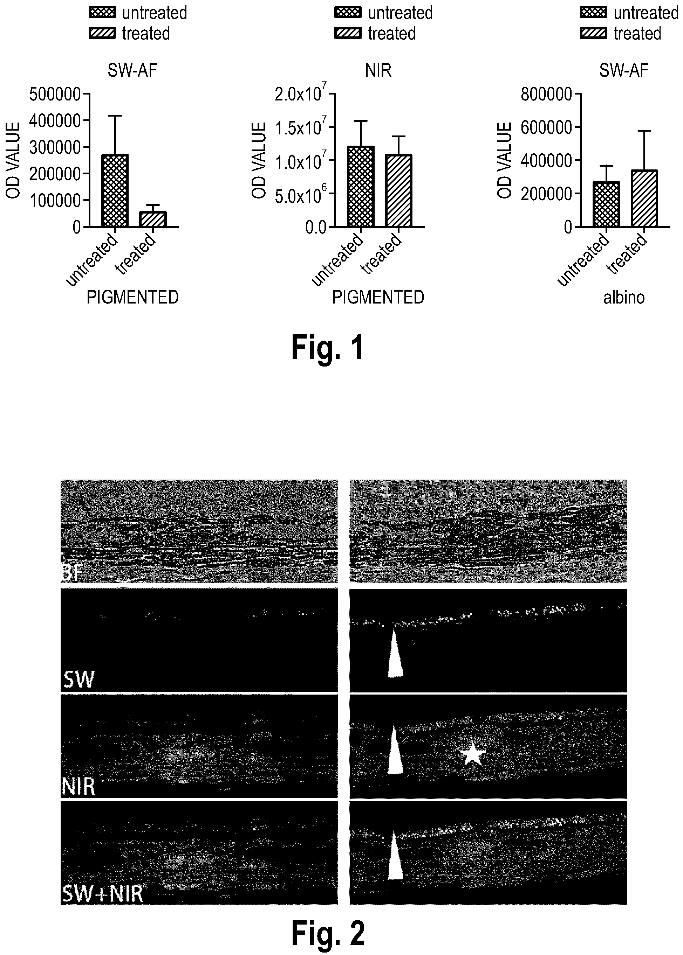

- Figure 1: In the pigmented mice there was a significant (p < 0.05) reduction of the lipofuscin content in the retinal pigment epithelium (RPE) as a mean value from 5 eyes compared to 5 untreated eyes which was recognized by the loss of SW-AF (Fig.1 left). The NIR-AF fluorescence of the melanosomes was also reduced but not significant (p > 0.05). (Fig.1 middle). In the albino mice SIN-1 had no effect on the fluorescence of lipofuscin (p > 0.05) (Fig.1 right).
- Figure 2: Semi thin sections through pigmented Abaca^{4-/-} mice eyes are shown. In the left column the effect of drug treatment becomes evident compared with a section from the untreated eye of the same mouse in the right column. The SW-AF image indicates that the lipofuscin granules (green) are reduced in number after treatment. Also the NIR-AF is somewhat diminished (red) in the RPE (arrowhead) but seems to be not effected in the melanosomes of the choroidal melanocytes (asterisk). The lower row shows a merged image of both fluorescence modalities.

### 1. Material and Methods

### Animals

Pigmented WT mice (129S2), pigmented Abca^{4-/-} mice (129S4/SvJae-Abca4tm1Ght) and albino Abca^{4-/-} mice^{(BALB/c-Abca4tm1Ght)} (Stargardt mice) were used in this work. WT mice were purchased from Harlan Laboratories (Hillcrest, UK), the knock-out strains were bred in the inventor's in-house facility. Light cycling was 12 h light (approximately 50 Ix in cages)/12 h dark, food and water were available ad libitum. Animals were anesthetized by i.p. injection of a mix of fentanyl (0.05mg/kg body weight), midazolam (5.0mg/kg body weight) and medetomidine (0.50mg/kg body weight).

### Drug treatment

130 µg of 3-Morpholinylsydnonimine chloride (SIN-1) dissolved in PBS was injected into the right eye of 5 pigmented and 5 albino Abca^{4-/-} mice. The mice were 2 years old. The left eyes remained untreated as controls. The mice were killed 48 hours after the injections and the eyes were emulated.

### Sample preparation for fluorescence and electron microscopy

Mouse eyes were fixed, embedded in epon resin and sectioned according to standard procedures. For fluorescence analysis, post-fixation and staining with heavy metals was omitted. Semi-thin sections (500 nm) were prepared and cover-slipped with Dako fluorescent mounting medium.

### Fluorescence microscopy

Specimens were investigated with a Zeiss Axioplan 2 microscope (Zeiss, Jena, Germany) equipped with a Lumencor Sola SE II NIR (Beaverton, OR, USA) light source and using a x63 objective. Filter sets were a custom made lipofuscin filter set (excitation 370/36 nm, emission 575/15, 400 nm beam splitter) for short wavelength-autofluorescence (SW-AF) and a commercial Cy7 filter set (excitation 708/75 nm, emission 809/81 nm, 757 nm beam splitter) for NIR-AF (Near infrared AF), respectively. The lipofuscin filter set is designed to fit the reported excitation and emission maximums for lipofuscin. Binning x2 was applied for all images and acquisition times, as well as microscope and software settings were held constant for any set of samples to allow comparison of fluorescence intensities. If indicated, images with low AF intensities were post-processed with the Auto adjust colors function of the IrfanView software to allow the localization of weak AF signals. This function enhances the contrast by redefining the darkest pixel as pure black and the brightest pixel as pure white in the histogram on a channel-by-channel basis.

### Semi-quantitative analysis of fluorescence intensities

Fluorescence intensities were determined using Fiji software. Regions of interest were determined in fluorescence and BF images using the default threshold, and area and integrated optical density (IOD) of the region of interest were measured. To correct for varied amounts of pigment granules displayed in each image, fluorescence IOD values were normalized to the area of pigment thresholded in corresponding BF images.

### Statistical analysis

Student's t-test with JMP 13 software (SAS, Cary, NC, USA) was used for statistical analysis. P < 0•05 was considered statistically significant.

### 2. Results

In pigmented mice there was a significant (p < 0.05) reduction of the lipofuscin content in the retinal pigment epithelium (RPE) as a mean value from 5 eyes compared to 5 untreated eyes which was recognized by the loss of SW-AF (Fig.1 and 2 left). The NIR-AF fluorescence of the melanosomes was also reduced but not significant (p > 0.05). (Fig. 1 middle). In the albino mice SIN-1 had no effect on the fluorescence of lipofuscin (p > 0.05) (Fig.1 right).

An original example of the fluorescence intensities after SIN-1 treatment and without is shown in semi thin sections through pigmented Abca^{4-/-} mice eyes in Fig. 2. In the left column the effect of drug treatment becomes evident compared with a section from the untreated eye of the same mouse in the right column. The SW-AF image indicates that the lipofuscin granules are reduced in number after treatment. Also the NIR-AF is somewhat diminished in the RPE (arrowhead) but seems to be not effected in the melanosomes of the choroidal melanocytes (asterisk). The lower row shows a merged image of both fluorescence modalities.

### 3. Conclusion

The inventor was surprisingly able to demonstrate that any of 3-morpholinosydnonimine (SIN-1), Rose Bengal (Acid Red 94), DEA-NO, isosorbide dinitrate (ISDN), isosorbide mononitrate (ISMN), sodium nitroprusside (SNP) and pentaerythritol tetranitrate (PETN) is capable to remove lipofuscin and/or aged oxidized melanin excess from a living being, in particular from the retinal pigment epithelium. The indicated compounds are, therefore, predestined for a therapeutic and/or prophylactic treatment of a living being affected by or suspected of being affected by a lipofuscin-associated disease and/or a disease associated with aged oxidized melanin.

## Claims

1. A compound for use in the treatment and/or prophylaxis of a lipofuscin-associated disease and/or a disease associated with aged oxidized melanin of a living being, which is selected from the group consisting of: 3-morpholinosydnonimine (SIN-1), Rose Bengal (Acid Red 94), DEA-NO, isosorbide dinitrate (ISDN), isosorbide mononitrate (ISMN), sodium nitroprusside (SNP), pentaerythritol tetranitrate (PETN), nitroglycerin (NG), iron (Fe), agents performing redox reactions with melanosomes, agents effecting chemiexcitation of melanin, and agents forming cyclobutane pyrimidine dimers (CPDs).

2. The compound of claim 1, **characterized in that** the lipofuscin-associated disease and/or the disease associated with aged oxidized melanin is a lipofuscin-associated disease of the eye and/or a disease of the eye associated with aged oxidized melanin.

3. The compound for use according to claim 1 or 2, **characterized in that** it is configured for an injection into the living being.

4. The compound according to any of the preceding claims, **characterized in that** it is configured for an intravenous and/or intravitreal injection into the living being.

5. The compound for use according to any of the preceding claims, **characterized in that** the lipofuscin-associated disease of the eye is selected from the group consisting of: retinal lipofuscinopathy, age-related macular degeneration (AMD), preferably in its dry form (dry AMD), Morbus Stargardt, vitelliform macular dystrophy (Best disease), and Retinitis pigmentosa (RP).

6. A pharmaceutical composition for the treatment and/or prophylaxis of a lipofuscin-associated disease and/or a disease associated with aged oxidized melanin of a living being comprising the compound of any of the claims 1 to 5 and a pharmaceutically acceptable carrier.

7. The pharmaceutical composition of claim 6, **characterized in that** the lipofuscin-associated disease and/or the disease associated with aged oxidized melanin is a lipofuscin-associated disease of the eye and/or a disease of the eye associated with aged oxidized melanin.

8. A method for the production of a pharmaceutical composition for the treatment and/or prophylaxis of a lipofuscin-associated disease and/or a disease associated with aged oxidized melanin of a living being, comprising the following steps:
(i) Providing the compound of any of claims 1 to 5, and
(ii) Formulating said compound into a pharmaceutically acceptable carrier.

9. The method of claim 8, **characterized in that** the lipofuscin-associated disease and/or the disease associated with aged oxidized melanin is a lipofuscin-associated disease of the eye and/or a disease of the eye associated with aged oxidized melanin.

10. A compound for use in the removal of lipofuscin and/or aged oxidized melanin from a living being, which is selected from the group consisting of: 3-morpholinosydnonimine (SIN-1), Rose Bengal (Acid Red 94), DEA-NO, isosorbide dinitrate (ISDN), isosorbide mononitrate (ISMN), sodium nitroprusside (SNP), pentaerythritol tetranitrate (PETN), nitroglycerin (NG), iron (Fe), agents performing redox reactions with melanosomes, agents effecting chemiexcitation of melanin, and agents forming cyclobutane pyrimidine dimers (CPDs).

11. The compound of claim 10 for use in the removal of lipofuscin and/or aged oxidized melanin from the eye of the living being.

12. The compound for use according to claim 11, **characterized in that** it is configured for a removal of the lipofuscin and/or aged oxidized melanin from the retinal pigment epithelium (RPE) of the living being.

13. A method for the therapeutic and/or prophylactic treatment of a living being affected by or suspected of being affected by a lipofuscin-associated disease and/or a disease associated with aged oxidized melanin, comprising the following steps:
(i) Providing the compound of any of claims 1 to 5 or the pharmaceutical composition of claim 6 or 7, and
(ii) administering said compound and/or said composition to said living being.

14. The method of claim 13, **characterized in that** the lipofuscin-associated disease and/or the disease associated with aged oxidized melanin is a lipofuscin-associated disease of the eye and/or a disease of the eye associated with aged oxidized melanin.
